**Europäisches Patentamt**

**European Patent Office**

**Office européen des brevets**

(19)

(11) Veröffentlichungsnummer : **0 206 997**
**B1**

(12) **EUROPÄISCHE PATENTSCHRIFT**

(45) Veröffentlichungstag der Patentschrift :
**17.05.89**

(21) Anmeldenummer : **86810269.0**

(22) Anmeldetag : **13.06.86**

(51) Int. Cl.⁴ : **A 61 M   5/14,** F 16 K   7/06,
B 26 B   27/00

(54) **Gerät zum Verschliessen und Trennen eines Schlauches, insbesondere eines bei einer Dialyse oder einer Infusion verwendeten Schlauches.**

(30) Priorität : **24.06.85 CH 2670/85**

(43) Veröffentlichungstag der Anmeldung :
**30.12.86 Patentblatt 86/52**

(45) Bekanntmachung des Hinweises auf die Patenterteilung : **17.05.89 Patentblatt 89/20**

(84) Benannte Vertragsstaaten :
**BE DE FR GB IT NL SE**

(56) Entgegenhaltungen :
**US–A– 3 323 208**
**US–A– 3 598 289**
**US–A– 3 631 858**
**US–A– 3 822 052**

(73) Patentinhaber : **Contempo Products, P. Herrli**
**Alpenstrasse 15a**
**CH-2502 Biel (CH)**

(72) Erfinder : **Herrli, Peter**
**Alpenstrasse 15a**
**CH-2502 Biel (CH)**

(74) Vertreter : **Schweizer, Hans et al**
**Bovard AG Patentanwälte VSP Optingenstrasse 16**
**CH-3000 Bern 25 (CH)**

EP 0 206 997 B1

Jouve, 18, rue St-Denis, 75001 Paris, France

## Beschreibung

Die Erfindung geht aus von einem Gerät zum Verschliessen und Trennen eines von einer Flüssigkeit durchgeflossenen elastisch nachgiebigen Schlauches, insbesondere eines bei einer Heimdialyse, Blutentnahme oder einer Infusion verwendeten Schlauches, mit einem ersten Teil zur Aufnahme des Schlauches, einem darin schwenkbar angeordneten zweiten Teil und einer am zweiten Teil befestigten Schneide zum Durchtrennen des Schlauches.

Dieses zweiteilige Gerät wird z. B. bei einer Heimdialyse, einer Blutentnahme oder einer Infusion verwendet. Ein Patient braucht beim Heimdialysatwechsel keinen Beutel mit frischem oder verbrauchtem Dialysat auf dem Körper zu tragen. In die Bauchhöhle des Patienten ist ein Katheter eingeführt, an welches sich ein Zwischenstück eines elastisch nachgiebigen Schlauches anschliesst. Mittels Metallkonnektoren wird das Ende des Y-förmigen Schlauches, der an dem Beutel mit frischem Dialysat und an dem Beutel mit verbrauchtem Dialysat angeschlossen ist, mit dem Zwischenstück verbunden. Nachdem der verbrauchte Dialysat aus dem Abdomem des Patienten in den Auslaufbeutel abgeführt wurde, muss der Y-förmige Schlauch verschlossen und anschliessend getrennt werden. Das Verschliessen wird durch Abklemmen erzielt, wodurch der Eintritt von Bakterien in den Abdomen des Patienten verhindert werden soll. Das Abtrennen wird dann durch eine Schere hervorgebracht.

Der voneinander getrennte Verschliess- und Trennvorgang verlief bis jetzt in fünf Arbeitsgängen, welche von dem Patienten erlernt werden mussten. Es ist aber immer häufig vorgekommen, dass die Bauchfelle der Patienten doch mit Bakterien infiziert wurden.

In der US-PS 3 822 052 ist eine von Hand betätigbare Klemmvorrichtung zur Kontrolle des Flüssigkeitsdurchflusses durch einen elastisch nachgiebigen Schlauch umschrieben. Die Vorrichtung besteht aus einem Kunststoffstreifen, der abgebogen werden kann, um einen Basisteil und einen über ihm liegenden Hebelarm zu bilden. Sowohl der Basisteil als auch der Hebelarm weisen gegenüberliegende Abstützungen auf, zwischen welchen der eingeführte Schlauch abgeklemmt wird. Die Abklemmung wird durch Einrasten des Hebelarmes in einem senkrecht stehenden Teil des Streifens erzielt. Durch das Ausschwenken des senkrecht stehenden Teiles kann der Hebelarm von dem senkrechten Teil gelöst und somit auch die Abklemmung des Schlauches aufgehoben werden. Diese Vorrichtung dient aber nur zum Abklemmen des Schlauches, sie ist keineswegs zum Trennen (Durchschneiden) des Schlauches ausgerüstet.

Das in der US-A 3 323 208 beschriebene Gerät zum gleichzeitigen Klemmen und Schneiden eines Schlauches umfasst einen ersten Teil und einen schwenkbar mit diesem verbundenen zweiten Teil gemäß dem Oberbegriff des Anspruchs 1.

Weil die am ersten Teil befestigte Messerklinge in der Ebene der Schwenkbewegung des ersten bzw. zweiten Teiles liegt, erstreckt sich der durchzuschneidende oder bereits durchgeschnittene und eingeklemmte Schlauch quer zur Längsachse des Gerätes. Dadurch beansprucht das Gerät mit dem darin eingeklemmten und sich quer dazu erstreckenden Schlauch relativ viel Platz. Zudem ist bei diesem Gerät nicht sichergestellt, dass der Schlauch nicht auf der falschen Seite abgetrennt wird. Ueberdies ist im geöffneten Zustand die Messerklinge frei zugänglich und es besteht deshalb eine erhebliche Veletzungsgefahr für den Benützer.

Weiter ist in der US-A 3 631 858 ein dreiteiliges Gerät zum Klemmen und Trennen der Nabelschnur eines neugeborenen Kindes beschrieben. Zwei der genannten Teile sind um je eine Welle schwenkbar mit dem dritten gemeinsamen Teil verbunden und sind in der Schliessstellung verriegelbar. Der eine erstgenannte und gemeinsame Teil weist je eine Klemmfläche auf, zwischen denen im geschlossenen Zustand des Gerätes die Nabelschnur eingeklemmt ist. An der Seitenfläche des anderen der beiden erstgenannten Teile ist eine Messerklinge befestigt, die zum Durchtrennen der Nabelschnur in Zusammenarbeit mit dem gemeinsamen Teil dient. Bei diesem Gerät sind zwei verschiedene Arbeitsgänge in der richtigen Reihenfolge auszuführen. Weiter haften diesem Gerät die gleichen Nachteile an, wie sie vorangehend mit Bezug auf das in der US-A-3 323 208 beschriebene Gerät angeführt sind.

Der im Anspruch 1 angegebenen Erfindung liegt die Aufgabe zugrunde, ein Gerät zum Verschliessen und Trennen eines von einer Flüssigkeit durchgeflossenen, elastisch nachgiebigen Schlauches zu schaffen, welches eine grössere Sicherheit als bisher für den Patienten bringen, indem kein offener Schlauchteil nach seinem Abtrennen verbleibt, was immer eine grosse Lebensgefahr für den Patienten darstellte. Es soll auch einem unangelernten Patienten das Verschliessen und Trennen von Hand ohne Mühe erlaubt werden, wobei auch die einzelnen Positionen des Bewegungsvorganges akustisch vernehmbar sein sollen. Durch das Gerät sollen die bisher verübten mehreren Arbeitsgänge eliminiert werden. Durch das Gerät soll weiter sichergestellt werden, dass der Schlauch nicht auf einer falschen Seite abgetrennt wird. Schlussendlich sollen die Herstellungskosten eines solchen Gerätes niedrig gehalten werden. Diese Aufgabe wird durch die Merkmale im kennzeichnenden Teil des Patentanspruches 1 gelöst.

Die Erfindung wird nachstehend anhand der Zeichnung beispielsweise näher erläutert. Es zeigen :

Fig. 1 eine Ansicht eines Teiles des Gerätes, welcher Teil an dem anderen, in der Figur 2 dargestellten Teil schwenkbar befestigbar ist,

Fig. 2 eine Ansicht des anderen Teiles des Gerätes,

Fig. 3 eine Seitenansicht des anderen Teiles nach der Figur 2,

Fig. 4 einen Schnitt entlang der Linie IV-IV der Figur 1,

Fig. 5 eine Seitenansicht der Federn nach der Figur 1 und

Fig. 6, 7, 8 Ansichten des aus zwei Teilen zusammengesetzten Gerätes in verschiedenen Einraststellungen.

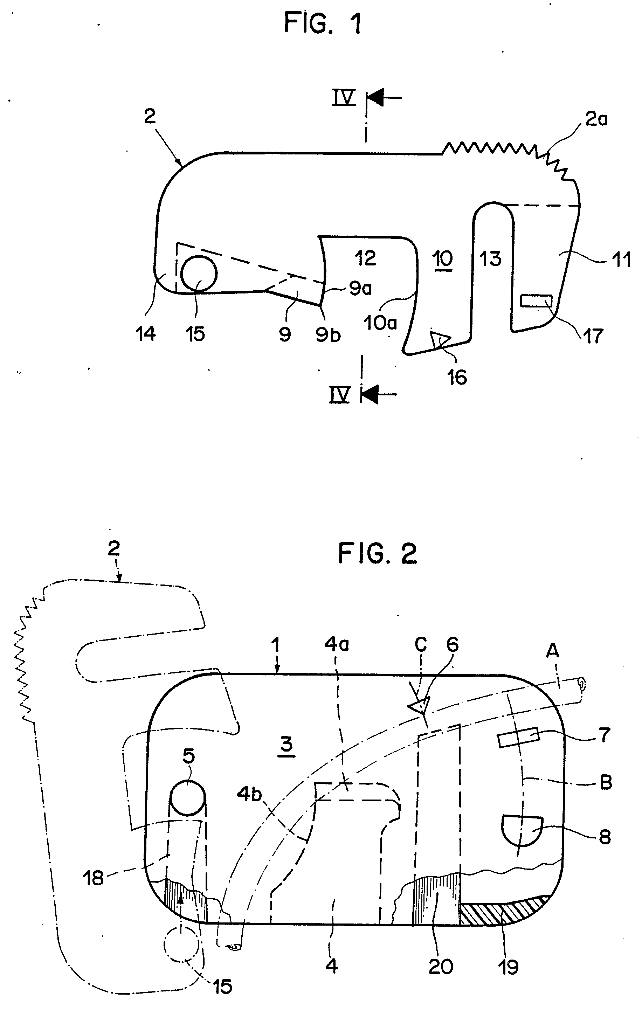

Der erste Teil 1 des zweiteiligen Gerätes nach der Figur 2 weist zwei zueinander parallel verlaufende viereckige Wände 3 auf, zwischen welchen ein Auflagestück 4 untergebracht ist. Das Auflagestück 4 bildet einerseits einen Teil eines der Längsränder des ersten Teiles 1 und andererseits reicht er bis etwa in die Mitte der Wandhöhe, so dass am anderen Längsrand des Teiles 1 zwischen den Wänden 3 ein Spalt zum Einlegen eines elastisch nachgiebigen Schlauches A zum Führen der Flüssigkeit entsteht. Zum Einlegen des Schlauches A dient eine am Auflagestück 4 vorgesehene Schlauchführungsrinne 4a. Der erste Teil 1 weist weiter eine Verstärkungsplatte 19 auf. In den beiden Wänden 3 sind vier gleichachsige Paare von Rasteröffnungen 5, 6, 7, 8 vorgesehen.

Der zweite Teil 2 nach der Figur 1 besitzt einen Lagerungsarm 14 mit einer eine Schneidekante 9b aufweisende Schneide 9, einen Klemmschnabel 10 und zwei Rastfedern 11. Zwischen der Schneide 9 und dem Klemmschnabel besteht eine erste Lücke 12 und zwischen dem Klemmschnabel 10 und den Rastfedern 11 eine zweite Lücke 13. Beidseitig des zweiten Teiles 2 sind drei gleichachsige Paare von Vorsprüngen 15, 16, 17 vorgesehen. Das erste Paar der Vorsprünge 15 befindet sich am Lagerungsarm 14, das zweite Paar 16 am Klemmschnabel 10 und das dritte Paar 17 an den Federn 11.

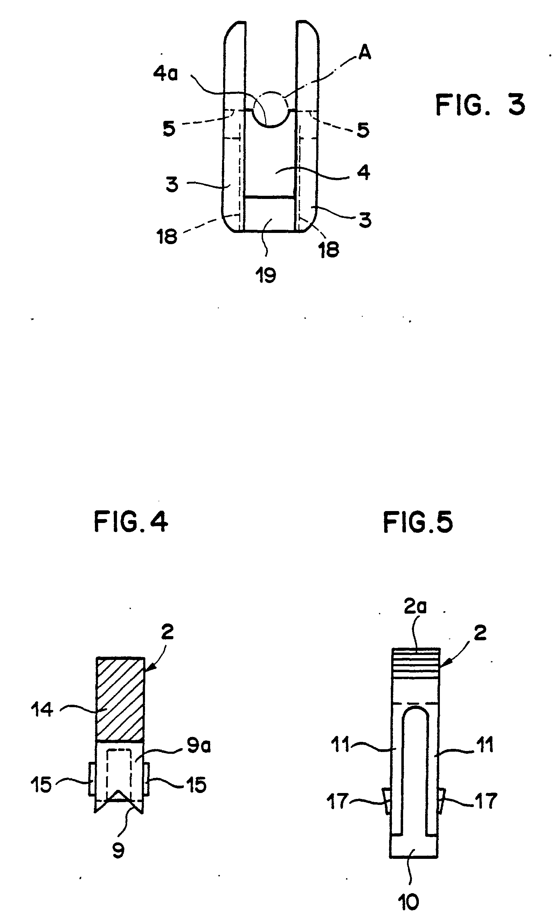

Bevor das erste Paar der Vorsprünge 15 in die Einlaufsnuten 18 und dann in das erste Paar der Rasteröffnungen 5 des ersten Teiles 1 eingeführt wird, wird in die Führungsrinne 4a des Auflagestückes 4 der Schlauch A (siehe Figur 3) so eingelegt, dass er auf der Auflagestückfläche 4b, die dem ersten Paar der Rasteröffnungen 5 zugekehrt ist, aufliegt. Nach dem Einführen des ersten Paares der Vorsprünge 15 in das erste Paar der Rasteröffnungen 5 ist der zweite Teil 2 mit dem ersten Teil 1 schwenkbar verbunden. Der Bewegungsvorgang des schwenkbaren zweiten Teiles 2 verläuft auf einer Kreisbogenlinie. Die Kreisbogenlinie des zweiten Paares der Vorsprünge 16 ist in der Figur 2 mit C und die Kreisbogenlinie des dritten Paares der Vorsprünge 17 mit B bezeichnet.

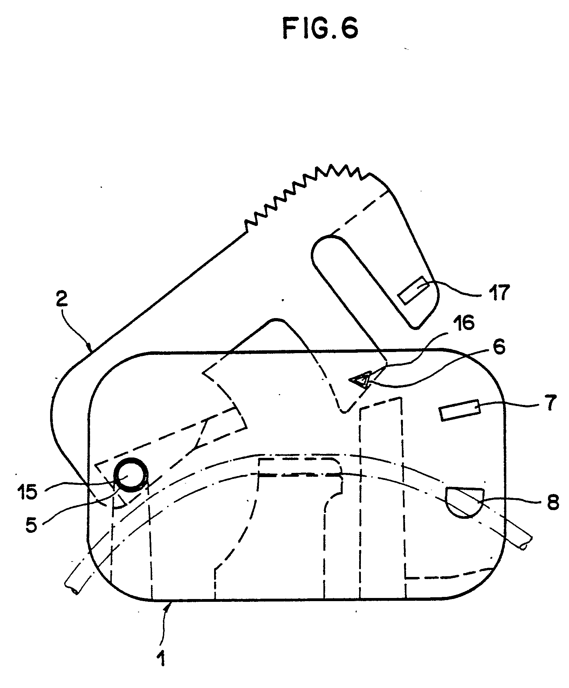

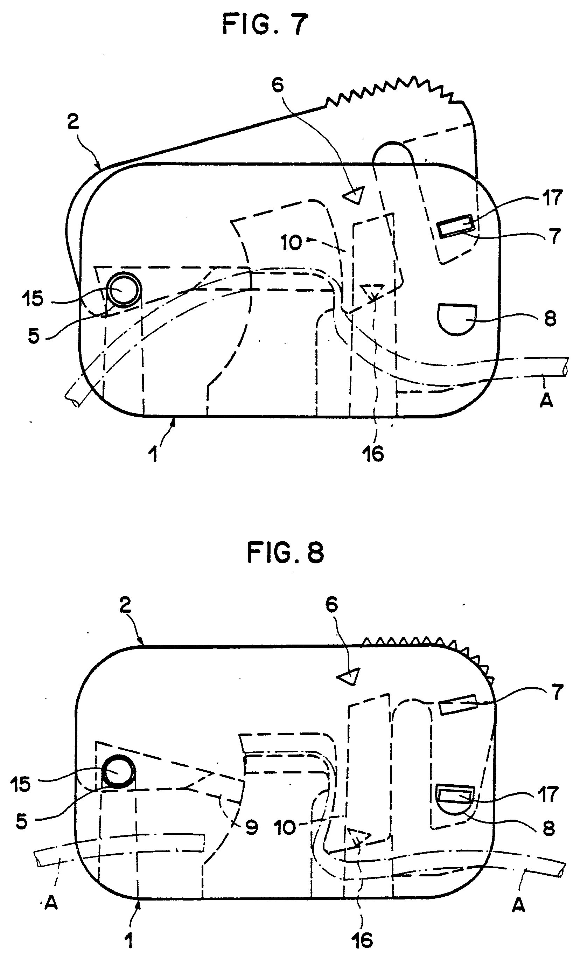

Im Laufe seiner Schwenkbewegung rastet das am Klemmschnabel 10 vorgesehene zweite Paar der Vorsprünge 16 in dem zweiten Paar der Rasteröffnungen 6 und das an den Rastfedern 11 vorgesehene dritte Paar der Vorsprünge 17 im dritten und vierten Paar von Rasteröffnungen 7, 8 sukzessiv ein. In der ersten Einraststellung 5, 15 und 6, 16 befindet sich das Gerät in Durchflussstellung, d. h., dass die Flüssigkeit durch den Schlauch A unbehindert durchfliessen kann (Fig. 6). Dem Patienten wird das Gerät in dieser ersten Einraststellung mit dem Schlauch und den Beuteln geliefert. In der zweiten Einraststellung 7, 17 wird der Schlauch A von dem Klemmschnabel 10 auf das Auflagestück 4 in Z-Form angedrückt und vollständig abgeklemmt, so dass durch diese Klemmstelle kein Medium (Flüssigkeit oder Luft) durchgehen kann (Fig. 7). Zwischen der zweiten 7, 17 und der dritten 8, 17 Einraststellung wird der Schlauch A durch die Schneide 9 durchgeschnitten (Fig. 8). Das Einrasten der Vorsprünge 16, 17 in den Rasteröffnungen 6, 7, 8 macht sich durch einen Klick-Laut hörbar.

Die Schneide 9 ist als Winkelklinge ausgebildet. Die der ersten Lücke 12 zugekehrte Schneidefläche 9a verläuft bogenförmig und entspricht dem bogenförmigen Verlauf der Auflagestückfläche 4b, die dem ersten Paar der Rasteröffnungen 5 zugekehrt ist. Durch diese Massnahme wird erreicht, dass zwischen der zweiten 7, 17 und der dritten 8, 17 Einraststellung der Schlauch A im Abstand von der Klemmstelle quer abgeschnitten wird.

Der Klemmschnabel 10, durch welchen der Schlauch A abgeklemmt wird, besteht aus vollem Material. Aus diesem Grunde kann er während des Bewegungsvorganges des zweiten Teiles 2 nicht seitlich federn, wie es der Fall bei den Federn 11 ist. Deswegen wird das zweite Paar der Vorsprünge 16 auf den inneren Flächen der Wände 3 in Führungsnuten 20 geführt. Die der ersten Lücke 12 zugekehrte Fläche 10a des Klemmschnabels 10 verläuft ebenfalls bogenförmig, wodurch das Festklemmen des Schlauches A noch vergrössert wird.

Um die einzelnen Rasteröffnungspaare 5, 6, 7, 8 und die ihnen entsprechenden Vorsprungspaare 15, 16, 17 auch visuell voneinander besser unterscheiden zu können, sind sie in voneinander unterschiedlichen geometrischen Formen und Farben ausgeführt. So sind die ersten Rasteröffnungspaare 5 kreisförmig, die zweiten Rasteröffnungspaare 6 dreieckig, die dritten Rasteröffnungspaare 7 viereckig und die vierten Rasteröffnungspaare 8 halbkreisförmig ausgebildet. Dementsprechend sind auch die zugehörigen Vorsprungspaare gestaltet. Wie aus der Figur 5 ersichtlich ist, ist das Vorsprungpaar 17 auf den Federn 11 so gestaltet, dass nach dem Einrasten desselben in den Rasteröffnungen 7 eine Wiederöffnung, d. h. Zurückschwenken des zweiten Teiles 2, ausgeschlossen ist. Dies gilt selbstverständlich auch für die Einraststellung 8, 17. Diese zwei Einraststellungen gewähren eine doppelte Sicherheit für den Patienten.

Das Gerät besteht aus nur zwei Teilen, die aus Hartkunststoff in einem Spritzgussverfahren hergestellt sind. Das Gerät hat eine flache, höchstens 1 cm dicke, 5 cm lange und 3 cm hohe Form. Eine solche Form des Gerätes kann vom Patienten angenehm an seinem Körper getragen werden. Durch eine Daumenriffelung 2a an der der zweiten Lücke 13 abgekehrten Randfläche des zweiten Teiles 2 wird der Bewegungsvorgang desselben erleichtert.

Die beiden Vorgänge, nämlich das Klemmen und das nachträgliche Durchschneiden des Schlauches werden durch ein einziges oben beschriebenes Gerät ausgeführt. Das Gerät ist so konstruiert, dass der Schlauch durch dasselbe nicht durchgezogen werden muss. Der Schlauch wird in die Führungsrinne des Auflegestückes längsweise eingelegt. Das Gerät ist ein Wegwerfgerät und kann also nicht wieder verwendet werden. Weil es aus zwei Teilen besteht, ist seine Fabrikation preisgünstig. Auch die Montage ist rationell, weil sie ohne Werkzeuge verläuft.

**Patentansprüche**

1. Gerät zum Verschliessen und Trennen eines von einer Flüssigkeit durchflossenen elastisch nachgiebigen Schlauches, insbesondere eines bei einer Heimdialyse, Blutentnahme oder einer Infusion verwendeten Schlauches, mit einem ersten Teil zur Aufnahme des Schlauches, einem darin schwenkbar angeordneten zweiten Teil und einer am zweiten Teil befestigten Schneide zum Durchtrennen des Schlauches, dadurch gekennzeichnet, dass die Schneidekante (9b) der Schneide (9) sich quer zur Ebene in der der zweite Teil schwenkbar ist, erstreckt, dass der erste Teil (1) zwei zueinander parallel verlaufende Wände (3) besitzt, zwischen welchen ein Auflagestück (4) für den Schlauch (A) untergebracht ist, dass in den Wänden (3) vier Paare von Rasteröffnungen (5, 6, 7, 8) vorgesehen sind, dass die Rasteröffnungen jedes Paares an gegenüberliegenden Stellen angeordnet sind, dass der zweite Teil (2) einen Lagerungsarm (14) mit der Schneide (9), einen Klemmschnabel (10) und zwei Rastfedern (11) besitzt, dass die Schneide (9) von dem Klemmschnabel (10) durch eine erste Lücke (12) und der Klemmschnabel (10) von den Rastfedern (11) durch eine zweite Lücke (13) getrennt sind, dass der Klemmschnabel (10) so ausgebildet ist, dass er beim Schwenken des zweiten Teiles (2) den Schlauch (A) zwischen dem Auflagestück (4) und ihm einklemmt, bevor die Schneide (9) den Schlauch durchschneidet, dass am zweiten Teil (2) drei Paare von Vorsprüngen (15, 16, 17) vorgesehen sind, dass die Vorsprünge jedes Paares an gegenüberliegenden Stellen des zweiten Teils angeordnet sind, dass der zweite Teil (2) mit seinem im ersten Paar von Rasteröffnungen (5) eingerasteten, am Lagerungsarm (14) vorgesehenen ersten Paar von Vorsprüngen (15) mit dem ersten Teil (1) schwenkbar verbunden ist und im Laufe seiner Schwenkbewegung mit dem am Klemmschnabel (10) vorgesehenen zweiten Paar von Vorsprüngen (16) in dem zweiten Paar von Rasteröffnungen (6) und mit dem an den Rastfedern (11) vorgesehenen dritten Paar von Vorsprüngen (17) im dritten und vierten Paar von Rasteröffnungen (7, 8) sukzessiv einrastbar ist.

2. Gerät nach Patentanspruch 1, dadurch gekennzeichnet, dass die der ersten Lücke (12)

zugekehrte Schneidefläche (9a) bogenförmig verläuft und der bogenförmig verlaufenden Auflagestückfläche (4b) entspricht, die dem ersten Paar der Rasteröffnungen (5) zugekehrt ist.

3. Gerät nach Patentanspruch 1, dadurch gekennzeichnet, dass die Schneide (9) als Winkelklinge gebildet ist.

4. Gerät nach Patentanspruch 1, dadurch gekennzeichnet, dass die der ersten Lücke (12) zugekehrte Fläche (10a) des Klemmschnabels (10) ebenfalls bogenförmig verläuft.

5. Gerät nach Patentanspruch 1, dadurch gekennzeichnet, dass die der zweiten Lücke (13) abgekehrte Randfläche (2a) des zweiten Teiles (2) geriffelt ist.

6. Gerät nach Patentanspruch 1, dadurch gekennzeichnet, dass die Rasteröffnungspaare (5, 6, 7, 8) und die ihnen entsprechenden Vorsprungspaare (15, 16, 17) in voneinander unterschiedlichen geometrischen Formen ausgeführt sind.

7. Gerät nach einem der Patentansprüche 1 bis 6, dadurch gekennzeichnet, dass es aus Hartkunststoff besteht und eine flache Form von höchstens 1 cm Dicke, 5 cm Länge und 3 cm Höhe hat.

**Claims**

1. Appliance for occluding and severing an elastically yielding tube traversed by a liquid, particularly a tube used in home dialysis, withdrawal of blood, or intravenous injection, having a first part for receiving the tube, a second part disposed pivotingly therein, and a cutter secured to the second part for cutting through the tube, characterized in that the cutting edge (9b) of the cutter (9) extends transversely to the plane in which the second part is pivoted, that the first part (1) has two walls (3) running parallel to one another, between which a supporting piece (4) for the tube (A) is accommodated, that four pairs of catch openings (5, 6, 7, 8) are provided in the walls (3), that the catch openings of each pair are disposed at opposite locations, that the second part (2) has a mounting arm (14) with the cutter (9), a clamping nose (10), and two catch springs (11), that the cutter (9) is separated from the clamping nose (10) by a first gap (12) and the clamping nose (10) from the catch springs (11) by a second gap (13), that the clamping nose (10) is so formed that upon pivoting of the second part (2) it pinches the tube (A) between the supporting piece (4) and itself before the cutter (9) cuts through the tube, that three pairs of projections (15, 16, 17) are provided on the second part (2), that the projections of each pair are disposed at opposite locations of the second part, that the second part (2) is pivotingly connected to the first part (1) by means of its first pair of projections (15) provided on the mounting arm (14) and snapped into the first pair of catch openings (5) and is successively snappable during the course of its pivoting movement into the second pair of catch openings (6) by means of the second pair of projections (16) provided on the clamping nose (10) and into the third and fourth pairs of catch openings (7, 8) by means of the third pair of projections (17) provided on the catch springs (11).

2. Appliance according to patent claim 1, characterized in that the cutting surface (9a) facing the first gap (12) runs arcuately and corresponds to the arcuately running supporting piece surface (4b) facing the first pair of catch openings (5).

3. Appliance according to patent claim 1, characterized in that the cutter (9) is formed as an angled blade.

4. Appliance according to patent claim 1, characterized in that the surface (10a) of the clamping nose (10) facing the first gap (12) likewise runs arcuately.

5. Appliance according to patent claim 1, characterized in that the edge surface (2a) of the second part (2) facing away from the second gap (13) is grooved.

6. Appliance according to patent claim 1, characterized in that the pairs of catch openings (5, 6, 7, 8) and the pairs of projections (15, 16, 17) corresponding thereto are executed in geometrical shapes different from one another.

7. Appliance according to one of the patent claims 1 to 6, characterized in that it is made of hard plastics and has a flat shape of at most 1 cm thickness, 5 cm length, and 3 cm height.

**Revendications**

1. Dispositif pour brider et couper un tube élastique et souple conduisant un liquide, en particulier un tube utilisé pour une dialyse à domicile, une prise de sang ou une perfusion, avec une première partie réceptrice du tube, une deuxième partie arrangée de manière à pouvoir pivoter, une lame tranchante destinée à couper le tube, étant fixée à cette deuxième partie, caractérisé en ce que le tranchant (9b) de la lame (9) est disposé transversalement au plan de la deuxième partie, en ce que la première partie (1) comprend deux parois parallèles entre elles (3) entre lesquelles une pièce de maintien (4) du tube (A) est disposée, en ce que, dans les parois (3), quatre paires d'ouvertures positionnées (5, 6, 7, 8) sont prévues telles que chaque paire d'ouvertures est prévue en position face à face, en ce que la deuxième partie (2) comprend un bras support (14) avec la lame (9), une pince en forme de bec (10) et deux lames ressorts (11), en ce que la lame (9) est séparée de la pince en forme de bec (10) par un premier évidement (12) et la pince en forme de bec (10) des lames ressorts (9) par un deuxième évidement (13), en ce que la pince en forme de bec (10) est ainsi faite que, en faisant pivoter la deuxième partie (2), le tube (A) est pincé entre la pièce de maintien (4) et elle-même, avant que la lame (9) ne coupe le tube, en ce que, sur la deuxième partie (2), trois paires de saillies (15, 16, 17) sont prévues, que chaque paire de saillies est positionnée sur la deuxième partie, que la deuxième partie (2) s'aligne sur la première paire d'ouverture (5) de la première partie ; sur le bras support (14), il est prévu que la première paire de saillies (15) est connectée de manière à pouvoir tourner dans la première partie (1) et que dans son mouvement de rotation avec la pince en forme de bec (10), il est prévu que la deuxième paire de saillies (16) s'enclenche dans la deuxième paire d'ouverture (6) et qu'ensuite, sur la lame ressort (11), il est prévu que la troisième paire de saillies (17) s'enclenche successivement dans les troisième et quatrième paire d'ouvertures (7, 8).

2. Dispositif selon la revendication 1, caractérisé en ce que, dans le premier évidement (12), la surface coupante (9a) est tournée en forme d'arc et que la surface de la pièce de maintien en forme d'arc (4b) lui correspond lorsque la première paire d'ouvertures (5) a tourné.

3. Dispositif selon la revendication 1, caractérisé en ce que la lame (9) est façonnée de forme angulaire.

4. Dispositif selon la revendication 1, caractérisé en ce que, dans le premier évidement (12), la surface pivotante (10a) de la pince en forme de bec (10) est pareillement façonnée en forme d'arc.

5. Dispositif selon la revendication 1, caractérisé en ce que la face opposée (2a) au deuxième évidement de la pièce (2) est striée.

6. Dispositif selon la revendication 1, caractérisé en ce que les paires d'ouvertures positionnées (5, 6, 7, 8) et les paires de saillies (15, 16, 17) qui leur correspondent sont taillées de forme géométrique différente.

7. Dispositif selon les revendications 1 à 6, dans lequel il est connu que le dispositif est en matière plastique dure, et a une forme plate de au plus 1 cm d'épaisseur, 5 cm de longueur et 3 cm de hauteur.

# FIG. 1

IV ◄

2          2a

12      10    13      11
9a
14  15    9  9b    10a      17
16

IV ◄

# FIG. 2

2

1    4a    C    6    A

3              7
5              B
4b
18              8

15    4    20    19

FIG. 3

4a

A

5 — — — — 5

3

4

3

18 — — — 18

19

FIG. 4

FIG. 5

2

2a

2

14

11 — — 11

9a

15 — 15

17 — 17

9

10

# FIG.6

## FIG. 7

## FIG. 8